# EUROPÄISCHE PATENTSCHRIFT

(11) **EP 3 679 347 B1**
(45) Veröffentlichungstag und Bekanntmachung des Hinweises auf die Patenterteilung: **17.07.2024**
(21) Anmeldenummer: 18762233.7
(22) Anmeldetag: 13.08.2018
(51) Int. Cl.: G01N 21/27, A61B 5/00, G01N 21/33, G01N 21/88, A45D 44/00, G01N 21/64, G01N 21/47

(54) **VERFAHREN UND VORRICHTUNG ZUM ERMITTELN EINES THERMISCHEN SCHÄDIGUNGSGRADS VON HAAR**
METHOD AND DEVICE FOR DETERMINING A DEGREE OF THERMAL DAMAGE TO HAIR
PROCÉDÉ ET DISPOSITIF POUR DÉTERMINER UN DEGRÉ DE DÉTÉRIORATION THERMIQUE DES CHEVEUX

(30) Priorität: 08.09.2017 DE 102017215873
(43) Veröffentlichungstag der Anmeldung: 15.07.2020
(73) Patentinhaber: Henkel AG & Co. KGaA, 40589 Düsseldorf (DE)
(72) Erfinder: MÜLLER, Burkhard, 40221 Düsseldorf (DE); LECHNER, Torsten, 40764 Langenfeld (DE)
(86) Internationale Anmeldenummer: PCT/EP2018/071928
(87) Internationale Veröffentlichungsnummer: WO 2019/048189

(56) Entgegenhaltungen:
- WO-A1-2011/024160
- DE-A1- 102016 225 674
- DE-A1- 19 506 677
- MCMULLEN R L ET AL: "Spectrofluorescent characterization of changes in hair chemistry induced by environmental stresses", J. COSMET. SCI. 62, 2011, pages 1 - 13, XP055896607, Retrieved from the Internet <URL:https://www.researchgate.net/profile/Roger-Mcmullen/publication/51186303_Spectrofluorescent_characterization_of_changes_in_hair_chemistry_induced_by_environmental_stresses/links/0deec539c6fbe0954d000000/Spectrofluorescent-characterization-of-changes-in-hair-chemistry-induced-by-environmental-stresse> [retrieved on 20220301]
- MCMULLEN R & JACHOWICZ J: "Thermal degradation of hair. I. Effect of curling irons", J. COSMET. SCI., 49, 1998, pages 223 - 244, XP055154260, Retrieved from the Internet <URL:http://journal.scconline.org/pdf/cc1998/cc049n04/p00223-p00244.pdf> [retrieved on 20141121]
- TATE M L ET AL: "Quantification and prevention of hair damage", J. SOC. COSMET. CHEM., vol. 44, 1993, pages 347 - 371, XP055513866, Retrieved from the Internet <URL:http://citeseerx.ist.psu.edu/viewdoc/download?doi=10.1.1.471.2274&rep=rep1&type=pdf> [retrieved on 20181004]
- JACHOWICZ J & MCMULLEN R L: "Tryptophan fluorescence in hair-Examination of contributing factors", J. COSMET. SCI., 62, 2011, pages 291 - 304, XP055511932, Retrieved from the Internet <URL:https://www.researchgate.net/profile/Roger_Mcmullen2/publication/51568946_Tryptophan_fluorescence_in_hair-Examination_of_contributing_factors/links/00b49539c6fede2bc7000000/Tryptophan-fluorescence-in-hair-Examination-of-contributing-factors.pdf> [retrieved on 20181002]
- CAO Y ET AL: "A novel method for non-destructive determination of hair photo-induced damage based on multispectral imaging technology", SCIENTIFIC REPORTS, vol. 7, no. 1, 31 March 2017 (2017-03-31), XP055512249, DOI: 10.1038/srep45544

## Beschreibung

Die Erfindung betrifft ein Verfahren und eine Vorrichtung zum Ermitteln eines thermischen Schädigungsgrads von Haar und ein Verfahren zum Ermitteln eines nutzerspezifischen Mittels zur Haarbehandlung.

Bei einer Behandlung von Haar mit kosmetischen Produkten kann eine Wirkung des Produkts, z.B. eine Intensität einer Färbung, stark vom Schädigungsgrad des Haars abhängen.

Deshalb kann eine Ermittlung einer Schädigung des Haars von großer Bedeutung sein.

Haar kann durch natürliche oder künstlich herbeigeführte Vorgänge geschädigt werden. Ein wichtiger Schädigungstyp kann dabei eine thermische Schädigung sein.

Beim Trocknen von feuchten Haaren mit einem Fön, aber insbesondere bei der semipermanenten Haarverformung mittels eines Glätteisens oder eines Lockenstabs, wirken hohe Temperaturen (bis 240 °C) auf das Haar ein. Insbesondere vorgeschädigtes Haar kann bei einem solch starken Hitzeeintrag, stark (weiter) geschädigt werden.

Der Schädigungsvorgang kann dabei ursächlich durch eine Zersetzung von Aminosäuren, beispielsweise eine Zersetzung der im Haar häufig vorkommenden Aminosäure Tryptophan und ihrer Oxidationsprodukte (Kynurenine), erfolgen.

Neben der chemischen Schädigung der Haare kann auch eine physikalische Schädigung auftreten, welche sich beispielsweise in der Ausbildung von Rissen in der Haarcuticula zeigt.

Diese strukturellen und chemischen Schädigungen des Haares können zu sprödem Haar, erhöhtem Haarbruch beim Kämmen und einer Erhöhung der Kämmarbeit führen.

Im akademischen und industriellen Bereich stehen einem Forscher bzw. Entwickler eine Vielzahl von physikalischen und chemisch-analytischen Verfahren zur Verfügung, um eine Ermittlung des thermischen Schädigungsgrads durchzuführen, beispielsweise eine quantitative Ermittlung des thermischen Schädigungsgrads.

Zur Bestimmung eines Gehalts einer Aminosäure können chromatographische Verfahren verwendet werden, wie beispielsweise Hochleistungsflüssigkeitschromatographie (HPLC). Diese erfordern aber zunächst einen aufwändigen sauren, basischen oder enzymatischen hydrolytischen Aufschluss der Haarprobe. Alternativ können colorimetrische Verfahren verwendet werden.

Allerdings sind all diese Verfahren kompliziert und apparativ aufwändig, so dass sie einem Endverbraucher nicht zur Verfügung stehen.

Schädigende kosmetische Behandlungen, beispielsweise Haarcolorationen, Hitzeanwendungen, Dauerwellen oder oxidative Prozeduren wie z.B. Blondieren, und viele andere mehr, werden typischerweise im privaten Bereich oder im Bereich kommerzieller Dienstleistungen am Endverbraucher ausgeführt. Obwohl eine Durchführung eines weiteren schädigenden Verfahrens an (thermisch) vorgeschädigtem Haar zu katastrophalen Ergebnissen bis hin zu einem vollständigen Haarbruch führen kann, bestand in diesem Rahmen bisher keine Möglichkeit, den Grad der thermischen Vorschädigung des Haars, beispielsweise quantitativ, zu bestimmen. Ferner wünschen sich immer mehr Anwender von Produkten ein auf ihre individuellen Bedürfnisse abgestimmtes Produkt. Dies gilt insbesondere auch für kosmetische Produkte wie Haut- und/oder Haarbehandlungsmittel. Dokument DE19506677 A1 beschreibt ein Verfahren zur Messung von Haareigenschaften unter Verwendung des vom Haar reemittierten Phosphoreszenzsignals. Dokument DE102016225674 A1 beschreibt ein Verfahren zur Messung von Haarschäden unter Verwendung von Interferenzmustern des vom Haar reflektierten Lichts. Dokument "Thermal degradation of hair. I. Effect ofcurling irons" by R. McMullen et. al. (J. Cosmet. Sci, 49 223-244, July/August 1998) beschreibt die Wirkung von Wärmebehandlungen auf das Haar mit Hilfe einer Fluoreszenzmessung. Dokument "Quantification and prevention of hair damage" by M.L. Tate et. al. (J. Soc. Cosmet. Chem. 44, 347-371 November/December 1993) beschreibt ein Verfahren zur Bewertung chemischer Haarschäden durch oxidative und reduktive Behandlungen. Dokument "Tryptophan fluorescence in hair-Examination ofcontributing factors" by Janusz Jachowicz, (J. Cosmet. Sci., 62, 291-304 May/June 2011) beschreibt Fluoreszenzmessungen am Haar und die Wirkung von Haartyp, Haarpigmentierung, chemischen Behandlungen und Foto- und Wärmeeinwirkung auf diese Spektren.

Erfindungsgemäß wird mit dem unabhängigen Anspruch 1 ein Verfahren zum Ermitteln eines thermischen Schädigungsgrads von Haar definiert, und wird mit dem unabhängigen Anspruch 10 eine Vorrichtung zum Ermitteln eines thermischen Schädigungsgrads von Haar definiert. Weitere vorteilhafte Ausgestaltungen ergeben sich aus den darauf rückbezogenen Unteransprüchen.

In verschiedenen Ausführungsbeispielen werden ein in der Nutzung einfaches Verfahren und eine entsprechende Vorrichtung bereitgestellt, welche mit Hilfe von UV-Spektroskopie oder UV/VIS-Spektroskopie eine präzise Bestimmung eines Grades an thermischer Schädigung von Haar ermöglichen.

In verschiedenen Ausführungsbeispielen kann aufgrund einer einfachen experimentellen Durchführung durch Nutzung neuartiger miniaturisierter UV-Sensoren oder UV/VIS-Sensoren eine mobile

Datenverarbeitungsvorrichtung, z.B. ein Smartphone, ein Tablet oder ein Laptop, für die Vorrichtung und das Verfahren genutzt werden.

In verschiedenen Ausführungsbeispielen wird ferner ein Verfahren zur Ermittlung individueller Haarbehandlungsanweisungen bereitgestellt, welches einem Endverbraucher auf einfache Weise erlaubt, den Grad der thermischen Schädigung, beispielsweise über den Gehalt an einer Aminosäure, in seinem Haar zu bestimmen und eine darauf abgestimmte Haarbehandlungsanweisung zu erhalten.

In verschiedenen Ausführungsbeispielen kann ein UV-Spektrum oder ein UV/VIS-Spektrum gewonnen werden.

Bei dem UV-Spektrum oder UV/VIS-Spektrum kann es sich beispielsweise um ein Absorptionsspektrum oder ein Fluoreszenzspektrum handeln.

Beispielsweise kann die thermische Haarschädigung in Form einer Abnahme des Gehalts an Tryptophan und/oder Kynureninen durch die Abnahme der für Tryptophan und Kynurenine charakteristischen Absorptionsbande bei 280 nm in einem Absorptionsspektrum bestimmt werden. Durch eine Anwendung von mathematischen Modellen kann mittels Vermessung von KalibrierHaarproben, welche einen anhand eines alternativen Verfahrens ermittelten thermischen Schädigungsgrad aufweisen, ein mathematisches Modell erstellt werden, welches dann bei einer Haarprobe, auch als Tresse bezeichnet, des Verbrauchers anhand des aufgenommenen UV- bzw. UV/VIS-Spektrums eine Berechnung eines thermischen Schädigungsgrad, beispielsweise eines Gehalts an Tryptophan und/oder Kynureninen, und damit der Haarschädigung, erlaubt. Eine Analyse des Spektrums und eine Anwendung des Modells kann dabei beispielsweise (mit geeigneten Apps) mittels eines Smartphones, eines Tablets, eines Smart Mirrors o.ä. ausgeführt werden.

Erfindungsgemäß wird ein Verfahren zum Ermitteln eines thermischen Schädigungsgrads von Haar bereitgestellt. Das Verfahren weist *inter alia* auf: während eines Belichtens einer Haarprobe des Haars mit UV- oder UV/VIS-Licht, Aufnehmen eines Spektrums von zumindest einem Teil des UV- oder UV/VIS-Lichts, welches mit der Haarprobe gewechseltwirkt hat, Vergleichen zumindest eines Teils des Spektrums mit einem mittels UV- oder UV/VIS-Spektren und thermischen Schädigungsgraden einer Mehrzahl von Kalibrierhaarproben gewonnenen spektroskopischen Kalibriermodell, und Ermitteln des thermischen Schädigungsgrads des Haars unter Einbeziehung des Vergleichs.

Dieses Verfahren erlaubt eine direkte, zerstörungsfreie Bestimmung eines thermischen Schädigungsgrads von Haaren, beispielsweise durch Bestimmung des Gehalts an Tryptophan und/oder anderen Bestandteilen in einer Haarprobe, ohne eine aufwändige Probenvorbereitung. Dieses Verfahren ermöglicht ein schnelleres Erzielen von Ergebnissen. Darüber hinaus kann es möglich sein, die Haare nach der Messung weiteren Behandlungen zu unterziehen, so dass an einer Haarsträhne Mehrfachanwendungen durchgeführt werden können.

Zudem kann es möglich sein, relativ leicht Messungen an verschiedenen Haarpositionen durchzuführen (z.B. kopfhautnahes und kopfhautentferntes Haar), beispielsweise auch direkt am Kopf, ohne die Haarprobe entnehmen zu müssen.

Ultraviolettstrahlung (UV-Strahlung), umgangssprachlich oft als ultraviolettes Licht (UV-Licht) bezeichnet, ist elektromagnetische Strahlung in einem Wellenlängenbereich von 10 bis 380 nm. Innerhalb dieses Wellenlängenbereiches wird zwischen "nahem UV" (UV-A) mit einem Wellenlängenbereich von 315 bis 380 nm, "mittlerem UV" (UV-B) mit einem Wellenlängenbereich von 280 bis 315 nm, "fernem UV" (UV-C-FUV) mit einem Wellenlängenbereich von 200 bis 280 nm, "Vakuum-UV" (UV-C-VUV) mit einem Wellenlängenbereich von 100 bis 200 nm und "extremem UV" (EUV) mit einem Wellenlängenbereich von 10 bis 121 nm unterschieden.

Im elektromagnetischen Spektrum umfasst der Bereich des sichtbaren Lichts (VIS) Wellenlängen von 380 nm bis 780 nm.

In verschiedenen Ausführungsbeispielen kann das UV-Licht oder UV/VIS-Licht, mit welchem die Haarprobe belichtet wird, einen Wellenlängenbereich von 200 bis 500 nm, insbesondere bevorzugt einen Wellenlängenbereich von 250 bis 400 nm und ganz besonders bevorzugt 250 bis 350 nm, aufweisen. Es kann insbesondere bevorzugt sein, dass das UV-Licht oder UV/VIS-Licht, mit welchem die Haarprobe belichtet wird, einen Wellenlängenbereich um etwa 280 nm, vorzugsweise zwischen 270 und 290 nm, aufweist.

Erfindungsgemäß weist das Verfahren ferner ein Erstellen des Kalibriermodells auf, wobei das Erstellen des Kalibriermodells *inter alia* aufweist: für die Mehrzahl von Kalibrierhaarproben ein Aufnehmen eines Kalibrierspektrums von zumindest einem Teil des UV- oder UV/VIS-Lichts, welches mit der Kalibrierhaarprobe wechselwirkt während eines Belichtens der Kalibrierhaarprobe mit UV- oder UV/VIS-Licht, ein Ermitteln eines thermischen Schädigungsgrads der Kalibrierhaarprobe mittels eines unabhängigen Verfahrens, und ein Zuweisen des thermischen Schädigungsgrads zum Kalibrierspektrum, und ein Ermitteln einer Korrelation zwischen der Mehrzahl von Kalibrierspektren und der Mehrzahl von thermischen Schädigungsgraden.

In verschiedenen Ausführungsbeispielen kann das unabhängige Verfahren zum Ermitteln eines thermischen Schädigungsgrads ein Ermitteln eines Tryptophangehalts und/oder eines Gehalts an und/oder Kynureninen aufweisen. Das unabhängige Verfahren kann ein chromatographisches oder colorimetrisches Verfahren sein. Das unabhängige Verfahren kann vorzugsweise ein chromatographisches Verfahren zur Bestimmung des Tryptophangehalts sein.

In diesen Ausführungsbeispielen kann es vorteilhaft sein, dass die Wechselwirkung eine Absorption von UV- oder UV/VIS-Licht umfasst. Hierbei kann es insbesondere bevorzugt sein, dass das UV-Licht oder UV/VIS-Licht, mit welchem die Haarprobe und/oder Kalibrierhaarprobe belichtet wird, einen Wellenlängenbereich um etwa 280 nm aufweist und dass der zumindest eine Teil des UV- oder UV/VIS-Lichts einen Wellenlängenbereich um etwa 280 nm aufweist.

Alternativ kann die Wechselwirkung eine Reflexion, Streuung, Transmission und/oder eine Fluoreszenz umfassen.

In verschiedenen Ausführungsbeispielen kann das Verfahren ferner ein Erstellen des Kalibriermodells aufweisen, wobei das Erstellen des Kalibriermodells aufweisen kann: für die Mehrzahl von Kalibrierhaarproben ein Aufnehmen eines Spektrums von zumindest einem Teil des UV- oder UV/VIS-Lichts, welches mit der Kalibrierhaarprobe wechselwirkt während eines Belichtens der Kalibrierhaarprobe mit UV- oder UV/VIS-Licht, ein Ermitteln eines Intensitätsverhältnisses der Intensität an UV- oder UV/VIS-Licht mit dem die Kalibrierhaarproben belichtet wurden zu der im Spektrum detektierten Intensität an UV- oder UV/VIS-Licht für jede detektierte Wellenlänge, ein Ermitteln eines thermischen Schädigungsgrads der Kalibrierhaarprobe mittels eines unabhängigen Verfahrens, und ein Zuweisen des thermischen Schädigungsgrads zum Kalibrierverhältnis, und ein Ermitteln einer Korrelation zwischen der Mehrzahl von Kalibrierverhältnissen und der Mehrzahl von thermischen Schädigungsgraden.

In verschiedenen Ausführungsbeispielen kann das unabhängige Verfahren zum Ermitteln eines thermischen Schädigungsgrads ein Ermitteln einer fiktiven Haarschädigungszeit aufweisen.

In diesen Ausführungsbeispielen werden Kalibrierhaarproben in einem Zeitreihenversuch für eine bestimmte Zeitdauer, beispielsweise 0 bis 240 Minuten, einer Temperatur größer 180 °C, vorzugsweise 200 °C, ausgesetzt. In definierten Zeitintervallen werden die Kalibrierhaarproben mit UV-Licht oder UV/VIS-Licht bestrahlt. Zumindest ein Teil des UV- oder UV/VIS-Lichts, welches mit den Kalibrierhaarproben gewechselwirkt hat, wird detektiert. Anschließend wird für jede detektierte Wellenlänge das Verhältnis der Intensität des eingestrahlten UV- oder UV/VIS-Licht zu der Intensität des detektierten UV- oder UV/VIS-Licht bestimmt. Dadurch wird ein, für den jeweiligen thermischen Schädigungsgrad charakteristisches Intensitätsverhältnis von eingestrahltem UV- oder UV/VIS-Licht zu reflektiertem und/oder emittiertem und/oder transmittierten und/oder gestreutem UV- oder VIS-Licht erhalten.

In verschiedenen Ausführungsbeispielen kann der zumindest eine Teil des UV- oder UV/VIS-Lichts, welches mit der Haarprobe und/oder der Kalibrierhaarprobe gewechselwirkt hat, einen Wellenlängenbereich von 200 bis 600 nm, mehr bevorzugt 300 bis 400 nm und ganz besonders bevorzugt 300 bis 350 nm aufweisen.

Erfindungsgemäß wird eine Vorrichtung zum Ermitteln eines thermischen Schädigungsgrads von Haar bereitgestellt. Die Vorrichtung umfasst *inter alia* kann eine UV-Lichtquelle oder UV/VIS-Lichtquelle zum Belichten einer Haarprobe des Haars mit UV- oder UV/VIS-Licht, ein Spektrometer zum Aufnehmen von zumindest einem Teil eines Spektrums von UV-oder UV/VIS-Licht, welches mit der Haarprobe gewechselwirkt hat, und eine Datenverarbeitungsvorrichtung mit einem Datenspeicher, in welchem ein mittels einer Mehrzahl von Kalibrierhaarproben gewonnenes spektroskopisches Kalibriermodell gespeichert ist, und mit einem Prozessor zum Vergleichen zumindest eines Teils des Spektrums mit dem spektroskopischen Kalibriermodell und zum Ermitteln des thermischen Schädigungsgrads des Haars unter Einbeziehung des Vergleichs.

Die UV-Lichtquelle kann beispielsweise das UV LEDs Lichtmodul "FluoroVu" der Firma Eigen Imaging Inc. sein.

In verschiedenen Ausführungsbeispielen können die UV-oder UV/VIS-Lichtquelle und das Spektrometer eine integrierte Einheit bilden.

Ein geeignetes miniaturisiertes UV/VIS-Spektrometer, welches in einem Wellenlängenbereich von 200 bis 850 nm arbeitet, ist beispielsweise von der Firma Ocean Optics, Inc., unter der Bezeichnungen USB2000+UV-VIS, erhältlich.

In verschiedenen Ausführungsbeispielen können die integrierte Einheit und/oder die Datenverarbeitungsvorrichtung mobile Geräte sein.

In verschiedenen Ausführungsbeispielen können die UV-oder UV/VIS-Lichtquelle und das Spektrometer in einer, vorzugsweise mobilen, Datenverarbeitungsvorrichtung, insbesondere einem Smartphone oder einem Tablet, integriert sein. Alternativ können die UV-oder UV/VIS-Lichtquelle und das Spektrometer eine integrierte Einheit bilden, welche, beispielsweise mittels geeigneter Stecker, physisch mit einer, vorzugsweise mobilen, Datenverarbeitungsvorrichtung verbunden werden kann.

In verschiedenen Ausführungsbeispielen wird ein Verfahren zum Ermitteln einer individuellen, das heißt nutzerspezifischen, Behandlungsanweisung in Abhängigkeit vom ermittelten thermischen Schädigungsgrad bereitgestellt.

Gemäß verschiedenen Ausführungsformen kann die individuelle Behandlungsanweisung die Empfehlung von Blondiermitteln und/oder Haarfärbemitteln und/oder Haarpflegeprodukten und/oder Haarstylingprodukten umfassen. Es ist insbesondere bevorzugt, dass die individuelle Behandlungsanweisung die Empfehlung von Haarpflegeprodukten und/oder Haarstylingprodukten umfasst.

Die Empfehlung kann dabei die Anzeige oder Ansage eines konkreten Produktnamens und/oder Produktbilds eines Blondiermittels und/oder Haarfärbemittels und/oder Haarpflegeprodukts und/oder Haarstylingprodukts umfassen. Alternativ kann die Empfehlung die Anzeige und/oder Ansage einer Produktlinie oder -serie (Name, Text und/oder Bild), insbesondere eine Blondiermittellinie/-serie und/oder Haarfärbemittellinie/-serie und/oder HaarpflegeproduktlinieZ-serie und/oder Haarstylingproduktlinie/-serie, eines Herstellers umfassen.

Es bevorzugt, dass das empfohlene Blondiermittel und/oder Haarfärbemittel und/oder Haarpflegeprodukt und/oder Haarstylingprodukt Inhaltsstoffe enthält, die auf den ermittelten thermischen Schädigungsgrad der Haare abgestimmt sind. Es ist insbesondere bevorzugt, dass das empfohlene Blondiermittel und/oder Haarfärbemittel und/oder Haarpflegeprodukt und/oder Haarstylingprodukt einen Hitzeschutz bewirkt. Ein Hitzeschutz kann beispielsweise durch Einsatz von filmbildenden Polymeren und/oder Polysiloxanen in den Haarbehandlungsprodukten erzielt werden. Als filmbildendes Polymer kann insbesondere Octylacrylamide / Acrylates / Butylaminoethyl Methacrylate Copolymer (INCI) eingesetzt werden. Geeignete Polysiloxane umfassen insbesondere PEG-14 Dimethicone (INCI).

Es ist deshalb bevorzugt, dass die empfohlenen Blondiermittel und/oder Haarfärbemittel und/oder Haarpflegeprodukte und/oder Haarstylingprodukte einen Inhaltsstoff ausgewählt aus der Gruppe bestehend aus filmbildenden Polymeren, Polysiloxanen und Mischungen daraus. umfassen.

In einer alternativen Ausführungsform des Verfahrens umfasst die individuelle Behandlungsanweisung die Empfehlung, für einen bestimmten Zeitraum von Blondierungen und/oder oxidativem Färben und/oder permanenten Verformungen und/oder Hitzebehandlungen Abstand zu nehmen. Diese individuelle Behandlungsanweisung kann insbesondere bei einer relativen Einstufung des thermischen Schädigungsgrads in den Bereichen "sehr stark" und/oder "stark" erfolgen.

In einer weiteren, vorteilhaften Ausführungsform des Verfahren besteht die individuelle Behandlungsanweisung darin, dem Individuum zum/vom Einsatz von Haarbehandlungsprodukten, die der Anwender des Verfahrens und/oder das Individuum anhand von QR-Codes, NFC-Chips, Barcodes oder RFID-Chips identifiziert, zu- oder abzuraten.

In dieser Ausführungsform des Verfahrens kann der Anwender des Verfahrens, beispielsweise ein Friseur oder eine beliebige Person am Verkaufspunkt von Haarbehandlungsmitteln, nach Ermittlung des thermischen Schädigungsgrad geeignete oder nicht geeignete Haarbehandlungsmittel über QR-Codes, NFC-Chips, Barcodes oder RFID-Chips, welche beispielsweise am Haarbehandlungsmittel selber oder am Ort dessen Aufbewahrung, beispielsweise am Regal im Friseurgeschäft oder im Verkaufspunkt von Haarbehandlungsmitteln, angebracht sind, ermitteln.

QR-Codes, NFC-Chips, Barcodes oder RFID-Chips ermöglichen, Informationen kabellos zu übertragen.

Als Barcode wird eine optoelektronisch lesbare Schrift bezeichnet, die aus verschieden breiten, parallelen Strichen und Lücken besteht. Die Daten in einem Strichcode werden mit optischen Lesegeräten, wie zum Beispiel Barcodelesegeräten (Scanner) oder Kameras, maschinell eingelesen und elektronisch weiterverarbeitet. Viele smarte Endgeräte weisen eine Software auf, die es ermöglicht, mit der Digitalkamera des smarten Endgeräts zu erfassen und dem Anwender die Code-Information sofort in dekodierter Form anzuzeigen.

Ein QR-Code ("Quick Response") ist ein zweidimensionaler Code, der aus einer quadratischen Matrix aus schwarzen und weißen Quadraten besteht, die die kodierten Daten binär darstellen. Üblicherweise verfügen smarte Endgeräte über eine eingebaute Kamera. Nach dem Fotografieren des QR-Codes wird mit Hilfe einer Software der QR-Code ausgelesen/interpretiert.

NFC-Chips und RFID-Chips sind Sender-Empfänger-Systeme. In diesem Fall muss mindestens ein Kommunikationspartner aktiv sein, also die Kommunikation anregen. Der andere Partner kann ein beispielsweise ein Chip ohne Energieversorgung sein. Dieser passive Teil wird auch Transponder (=Transmitter&Responder) genannt. Neben der aktiv-passiv Kommunikation zwischen beispielsweise einem smarten Endgerät als aktivem Kommunikationspartner und einem Transponder/Chip ist ebenfalls eine aktiv-aktiv Kommunikation möglich.

Die Kopplung/Anregung geschieht durch vom aktiven Kommunikationspartner erzeugte magnetische Wechselfelder mit geringer Reichweite oder durch hochfrequente Radiowellen. Damit werden nicht nur Daten übertragen, sondern auch der Transponder mit Energie versorgt. Der aktive Kommunikationspartner, beispielsweise ein smartes Endgerät, enthält eine Software, die den eigentlichen Leseprozess steuert, und eine sogenannte Middleware mit Schnittstellen zu weiteren (mobilen) Datenverarbeitungsvorrichtungen und/oder Datenbanken.

RFID ("radio-frequency identification") funktioniert über Radiowellen. Die RFID-Technik umfasst ein sehr breites Angebot an verschiedenen Chips und Lesegeräten, welche sich im Wesentlichen durch Speicherkapazität, Herstellverfahren, Kosten, Frequenzbereich und durch die Reichweite unterscheiden.

NFC ("Near Field Communication") ist eine genormte Spezialisierung der RFID-Technik, die speziell für eine Datenübertragung über kurze Distanzen (max. 10 cm) entwickelt wurde.

QR-Codes, NFC-Chips, Barcodes oder RFID-Chips können beispielsweise Informationen enthalten, für welche thermischen Schädigungsgrade das zugehörige Haarbehandlungsmittel geeignet oder nicht geeignet ist.

Das Verfahren zum Ermitteln einer individuellen Behandlungsanweisung kann ferner auch das Einleiten eines online-Bestellvorgangs des ermittelten Haarbehandlungsprodukts (Blondiermittel und/oder Haarfärbemittel und/oder Haarpflegeprodukt und/oder Haarstylingprodukt) und/oder das Bereitstellen eines Hinweises, wo das ermittelte Haarbehandlungsprodukt (Blondiermittel und/oder Haarfärbemittel und/oder Haarpflegeprodukt und/oder Haarstylingprodukt) erhältlich ist, umfassen.

In einer alternativen Ausführungsform des Verfahrens besteht die individuelle Behandlungsanweisung darin, dem Nutzer zum Einsatz von Blondiermitteln und/oder Haarfärbemitteln und/oder Haarpflegeprodukten und/oder Haarstylingprodukten, die für den Nutzer individuell hergestellt werden, zu zuraten und einen Bestellvorgang, vorzugsweise durch Aufrufen einer Internetseite eines Herstellers von individuellen Blondiermitteln und/oder individuellen Haarfärbemitteln und/oder individuellen Haarpflegeprodukten und/oder individuellen Haarstylingprodukten, einzuleiten.

Immer mehr Kunden wünschen sich ein individuell auf ihre Bedürfnisse abgestimmtes Produkt. Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Bei Blondiermitteln, Haarfärbemitteln, Haarpflegeprodukten und Haarstylingmitteln umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten thermischen Schädigungsgrad ungeeigneter Inhaltsstoffe vermieden werden. Umgekehrt kann die Auswahl für den ermittelten thermischen Schädigungsgrad geeigneter Inhaltsstoffe durch den Produktkonfigurator vorgegeben oder vorgeschlagen werden.

In verschiedenen Ausführungsbeispielen kann bei bestimmten thermischen Haarschädigungsgraden der Besuch eines Friseurs empfohlen werden. In verschiedenen Ausführungsbeispielen kann direkt über die Software/App, welche den thermischen Schädigungsgrad ermittelt, ein Buchungsvorgang eingeleitet werden. Dazu können beispielsweise in der Software/App die Kontaktdaten von Friseuren hinterlegt sein und diese dem Nutzer angezeigt werden. Zusätzlich kann über Filter, wie beispielsweise die Postleitzahl, die Auswahl eingeschränkt werden. Alternativ kann die Buchung eines Friseurtermins über eine separate Software/App, wie beispielsweise Treatwell, erfolgen.

Entsprechend kann die individuelle Behandlungsanweisung die Empfehlung eines Friseurtermins und die Einleitung eines Terminbuchungsvorgangs umfassen.

Es ist auch bevorzugt, dass die individuelle Behandlungsanweisung gespeichert wird und in nachfolgenden Verfahren für eine langfristige Empfehlung genutzt wird.

In einer weiteren Ausführungsform findet vor der Ausgabe der individuelle Behandlungsanweisung an den Nutzer ein Datenabgleich zwischen der (mobilen) Datenverarbeitungsvorrichtung, insbesondere einem Smartphone oder Tablet, und Daten, die in einer Cloud hinterlegt sind, statt. Diese Daten können beispielsweise Daten von Nutzern mit gleichen oder ähnlichen thermischen Schädigungsgraden sowie gegebenenfalls weiteren gleichen oder ähnlichen Haar-Parametern und den daraus abgeleiteten Empfehlungen/Maßnahmen und Erfahrungswerte hinsichtlich eines Behandlungserfolgs sein. Durch Aktualisierung der Daten in der Cloud durch Aufnehmen von Erfahrungswerten weiterer Nutzer und Einbeziehung der Daten in das Verfahren zum Ermitteln einer individuellen Behandlungsanweisung kann für den Nutzer stets eine optimale Behandlungsanweisung ermittelt werden.

In verschiedenen Ausführungsbeispielen können beim Ermitteln der individuellen Behandlungsanweisung ferner Informationen hinsichtlich eines generellen Gesundheitszustands, Ernährungsgewohnheiten und weiterer Verhaltensweisen des Nutzers (z.B. tägliche Aufenthaltsdauer im Freien/in der Sonne/im Wasser, Rauchgewohnheiten, Sportgewohnheiten, usw.) verwendet werden.

Das Ermitteln des thermischen Schädigungsgrads des Haars und/oder Ermitteln der individuellen Behandlungsanweisung kann beispielsweise mit einer App/Software auf einem Tablet, einem Laptop, einem Smart Mirror oder einem Smartphone erfolgen. Entsprechend kann die App eine "mobile App" (Anwendungssoftware für Mobilgeräte), eine Web-App (Anwendungssoftware nach dem Client-Server-Modell) oder eine Desktop-App (Anwendungssoftware für einen Desktop-Computer) sein.

In verschiedenen Ausführungsbeispielen kann die Software/App, welche den thermischen Schädigungsgrad ermittelt, dieselbe sein, die die individuelle Behandlungsanweisung ermittelt. In verschiedenen Ausführungsbeispielen können unterschiedliche Softwareprogramme/Apps verwendet werden für einen Teil der verschiedenen Vorgänge oder alle verschiedenen Vorgänge.

Ausführungsbeispiele der Erfindung werden unter anderem anhand einer Figur dargestellt und werden im Folgenden näher erläutert.

Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

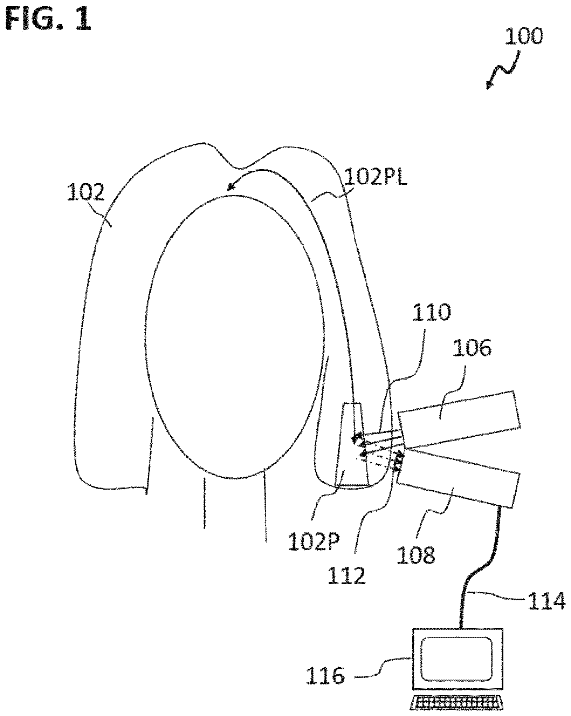

FIG. 1 zeigt in einer Ansicht 100 eine schematische Darstellung eines Verfahrens zum Ermitteln eines thermischen Schädigungsgrads von Haar gemäß verschiedenen Ausführungsbeispielen.

Gemäß verschiedenen Ausführungsbeispielen kann zum Ermitteln eines thermischen Schädigungsgrads von Haar 102 eines Nutzers eine Haarprobe 102P untersucht werden. Die Haarprobe 102P kann dabei am Kopf belassen oder entfernt werden. Die Haarprobe 102P kann sich in einer Entfernung 102PL von einer Kopfhaut des Nutzers befinden bzw. von dort entnommen werden. Die Haarprobe 102P kann eine Mindestmenge von Haaren aufweisen, die beispielsweise ausgedrückt sein kann als eine minimale Fläche, welche flächendeckend mit der (z.B. flach ausgebreiteten) Haarprobe 102P abgedeckt sein kann, beispielsweise mindestens 1 cm², oder beispielsweise als ein Mindestgewicht, beispielsweise mindestens 0,5 g.

In verschiedenen Ausführungsbeispielen kann zum Ermitteln des thermischen Schädigungsgrads des Haars 102 eine Vorrichtung genutzt werden, wie sie in FIG. 1 beispielhaft schematisch in einer Ansicht 100 dargestellt ist.

In verschiedenen Ausführungsbeispielen kann die Haarprobe 102P so ausgebreitet werden, dass sie zumindest einen Wechselwirkungsbereich bedeckt, welcher von einer Lichtquelle 106 mit UV-oder UV/VIS-Licht 110 beleuchtet wird und aus welchem das Licht 110, nach einer Wechselwirkung mit der Haarprobe 102 als zu analysierendes Licht 112 bezeichnet, in ein Spektrometer 108 eintritt.

In verschiedenen Ausführungsbeispielen kann die Haarprobe 102P mit UV-Licht 110 oder mit UV/VIS-Licht 110 beleuchtet werden. Das UV-Licht 110 kann einen Wellenlängenbereich von 200 bis 380 nm abdecken, oder mindestens einen geeigneten Teilbereich daraus, beispielsweise von 250 bis 300 nm. Das UV/VIS-Licht 110 kann einen Wellenlängenbereich von 200 bis 500 nm abdecken, oder mindestens einen geeigneten Teilbereich daraus, beispielsweise von 250 bis 400 nm.

In verschiedenen Ausführungsbeispielen kann eine Lichtquelle 106 des UV- oder des UV/VIS-Lichts 110 beispielsweise eine UV-Lampe oder eine UV/VIS-Lampe sein, oder jede andere herkömmliche Lichtquelle, die ein geeignetes Lichtspektrum bereitstellt.

Dank einer fortschreitenden Verbesserung von UV- oder UV/VIS-Spektroskopievorrichtungen können diese in miniaturisierter, z.B. mobiler, Form bereitgestellt werden, beispielsweise als eine Einheit, welche die Lichtquelle 106 und das Spektrometer 108 als integrierte Bestandteile aufweisen kann.

In verschiedenen Ausführungsbeispielen kann das Spektrum an eine Datenverarbeitungsvorrichtung 116 übertragen werden. Die Datenübertragung ist mit dem Bezugszeichen 114 gekennzeichnet. Die Übertragung kann auf bekanntem Weg erfolgen, beispielsweise mittels eines Datenkabels (zum Beispiel USB), kabelloser Datenübertragung (z.B. Bluetooth, WLan (WiFi), Thread, ZigBee oder Near Field Communication (NFC)), oder eine Übertragung kann innerhalb einer Vorrichtung erfolgen, wenn das Spektrometer (und ggf. die Lichtquelle) Teil einer Datenverarbeitungsvorrichtung (z.B. Smartphone, Tablet, Laptop oder Smart Mirror) ist oder an der Datenverarbeitungsvorrichtung lösbar (beispielsweise mittels eines geeigneten Steckers) angebracht ist oder umgekehrt das Spektrometer 108 mit einer integrierten Datenverarbeitungsvorrichtung 116 gebildet ist.

Zum Empfangen und Weiterverarbeiten der Daten und für die Modellbildung kann die Datenverarbeitungsvorrichtung in verschiedenen Ausführungsbeispielen mit einer entsprechenden Software ausgestattet sein, beispielsweise einer App.

In verschiedenen Ausführungsbeispielen kann anhand des aufgenommenen UV- oder UV/VIS-Spektrums in Verbindung mit dem Kalibrationsmodell ein Tryptophangehalt der Haarprobe wie oben beschrieben ermittelt werden.

In verschiedenen Ausführungsbeispielen kann anhand des aufgenommenen UV- oder UV/VIS-Spektrums in Verbindung mit dem Kalibrationsmodell eine fiktive Schädigungszeit der Haarprobe wie oben beschrieben ermittelt werden.

In verschiedenen Ausführungsbeispielen kann der thermische Schädigungsgrad in einer kategorialen Skala (z.B. gering, mäßig, stark, sehr stark) ermittelt werden.

In verschiedenen Ausführungsbeispielen kann der thermische Schädigungsgrad in einer metrischen Skala (z.B. ein Zahlenwert mit willkürlichen Einheiten, als Prozentanteil des Gehalts an Tryptophan oder als Prozentanteil an Schädigung) ermittelt werden.

In verschiedenen Ausführungsbeispielen kann das beschriebene Verfahren zum Ermitteln eines thermischen Schädigungsgrads von Haar mittels einer Datenverarbeitungsvorrichtung 116 ausgeführt werden.

Die Datenverarbeitungsvorrichtung kann, wie oben im Zusammenhang mit FIG. 1 beschrieben, beispielsweise eine mobile Datenverarbeitungsvorrichtung, beispielsweise ein Smartphone, ein Tablet oder einen Laptop, aber auch einen sonstigen Computer, zum Beispiel einen Smart Mirror, aufweisen, oder jede andere Datenverarbeitungsvorrichtung, die geeignet ist, die Daten zu speichern und bereitzustellen, den Vergleich auszuführen und das Modell anzuwenden, ggf. auch das Modell zu erstellen, also beispielsweise jede Datenverarbeitungsvorrichtung mit hinreichend großem Datenspeicher und hinreichend leistungsfähigem Prozessor.

In verschiedenen Ausführungsbeispielen kann die Datenverarbeitungsvorrichtung mindestens eine Eingabevorrichtung zum Eingeben von Informationen in die Datenverarbeitungsvorrichtung aufweisen, beispielsweise zum Eingeben von Tryptophangehalt-Messwerten für die Kalibrierung und ggf. zum Eingeben von Anweisungen, Parametern, Nutzerdaten usw. für ein Ausführen des Verfahrens.

In verschiedenen Ausführungsbeispielen kann die mindestens eine Eingabevorrichtung einen einen berührungsempfindlichen Bildschirm, ein Mikrofon und/oder eine Tastatur aufweisen.

In verschiedenen Ausführungsbeispielen kann die Datenverarbeitungsvorrichtung mindestens eine Ausgabevorrichtung zum Ausgeben von Informationen aufweisen, beispielsweise zum Ausgeben von Ergebnissen des Verfahrens.

In verschiedenen Ausführungsbeispielen kann die mindestens eine Ausgabevorrichtung einen (berührungsempfindlichen) Bildschirm, Lautsprecher und/oder einen Drucker aufweisen.

Insbesondere in einem Fall, in welchem der thermische Schädigungsgrad für eine Mehrzahl von Haarbereichen (zum Beispiel am Ansatz, in der Mitte und/oder an den Spitzen) ermittelt wird, kann das Bereitstellen des thermischen Schädigungsgrads an den Nutzer eine graphische Darstellung aufweisen, beispielsweise eine Anzeige, z.B. mittels einer Anzeigevorrichtung als Ausgabevorrichtung (z.B. eines (berührungsempfindlichen) Bildschirms eines Smartphones, Tablets oder Smart Mirrors). In der graphischen Darstellung kann der ermittelte thermische Schädigungsgrad der Mehrzahl von Bereichen mit einer Codierung anhand des thermische Schädigungsgrads in einer Darstellung des Nutzers (z.B. einer schematischen Darstellung oder auf einem Foto des Nutzers) angezeigt werden. Beispielsweise können in einer schematischen Darstellung der Frisur des Nutzers Bereiche unterschiedlichen thermische Schädigungsgrads mit unterschiedlichen Farben und/oder Mustern dargestellt werden, z.B. Bereiche mit niedrigem thermische Schädigungsgrad grün und Bereiche mit hohem thermische Schädigungsgrad rot oder ähnliches. In einem anderen Beispiel können einem Foto des Nutzers, z.B. einem digitalen Foto, welches die Haare oder Frisur des Nutzers zeigt, unterschiedliche Muster überlagert sein, z.B. ein Punktmuster für Bereiche mit hohem thermischen Schädigungsgrad und ein Linienmuster für Bereiche mit niedrigem thermische Schädigungsgrad oder ähnliches. Alternativ kann die Echtzeit-Darstellung der Haare auf/in einem Smart Mirror zur Darstellung der ermittelten thermischen Schädigungsgrade verwendet werden.

Beim graphischen Darstellen des thermische Schädigungsgrads des mindestens einen Haarbereichs, z.B. der Mehrzahl der Haarbereiche, kann in verschiedenen Ausführungsbeispielen nur für den Bereich/die Bereiche, für den/die der thermische Schädigungsgrad(e) ermittelt wurde(n), der thermische Schädigungsgrad dargestellt werden. In verschiedenen Ausführungsbeispielen kann ein Bereich, für den ein thermische Schädigungsgrad angezeigt wird, über den Haarbereich hinaus, für den der thermische Schädigungsgrad ermittelt wurde, extrapoliert werden, beispielsweise unter Einbeziehung typischer Schädigungsgradverteilungsmuster.

Zusätzlich oder alternativ können dem Nutzer mit Hilfe einer Anzeigevorrichtung als Ausgabevorrichtung konkrete Behandlungsanweisungen als Bilder und/oder Textnachrichten dargestellt werden. Beispielsweise können auf seinen thermischen Schädigungsgrad abgestimmte Abbildungen von konkreten Produkten angezeigt werden.

In verschiedenen Ausführungsbeispielen kann die mindestens eine Ausgabevorrichtung einen Lautsprecher und/oder die mindestens eine Eingabevorrichtung einen Lautsprecher In dieser Ausführungsform erfolgt Ausgabe des ermittelten Schädigungsgrades und/oder der Behandlungsanweisung als verbale Mitteilung. Die Eingabe von Informationen und/oder Sprachbefehlen erfolgt über die Stimme des Nutzers. Im diesen Fall weist die Vorrichtung ein Modul für Spracherkennung, vorzugsweise einen Intelligenten Persönlichen Assistenten (Sprachassistenten), wie beispielsweise Alexa von Amazon, Google Assistant von Google, Cortana von Microsoft oder Siri von Apple, auf.

In verschiedenen Ausführungsbeispielen kann die Vorrichtung In verschiedenen Ausführungsbeispielen kann für eine Modellierung jedes beliebige Programm (z.B. eine App) genutzt werden, welches eine solche Funktionalität bereitstellt.

Beispiel 1 außerhalb des Umfangs der angehängten Ansprüche, jedoch hilfreich für das Verständnis der Ansprüche

### 1. Spektroskopisches Kalibriermodell ("fiktive Haarschädigungszeit")

Zum Ermitteln eines spektroskopischen Kalibriermodells werden zunächst Haarsträhnen einer bestimmten Breite, beispielsweise 5 mm, in einem Zeitreihenversuch für 0 bis 120 Minuten einer Hitze von 200 °C ausgesetzt. In Intervallen von 10 Minuten wird jede Haarsträhne mit UV-Licht mit einer Wellenlänge von 250 bis 300 nm bestrahlt. Das Licht, welches mit den Haarsträhnen gewechselwirkt hat, wird in einem Wellenlängenbereich von 300 bis 350 nm detektiert. Anschließend wird für den Bereich von 300 bis 350 nm das Verhältnis von eingestrahlten zu detektiertem UV-Licht bestimmt. Dadurch wird ein, für den jeweiligen thermischen Schädigungsgrad charakteristisches Verhältnis von eingestrahltem UV-Licht zu reflektiertem und/oder emittiertem und/oder transmittierten und/oder gestreutem UV-Licht erhalten. Für jede Schädigungsdauer werden 10 Haarsträhnen vermessen und die erhaltenen Werte über den Wilcoxon-Vorzeichen-Rang-Test gemittelt.

### 2. Bestimmung des thermischen Haarschädigungsgrad

Zur Bestimmung einer individuellen Haarschädigung wird mindestens eine Haarsträhne, umfassend rund 100 Haare, eines Individuums mit UV-Licht mit einer Wellenlänge von 250 bis 300 nm bestrahlt, das Licht, welches mit den Haarsträhnen gewechselwirkt hat, in einem Wellenlängenbereich von 300 bis 350 nm detektiert und das Verhältnis von eingestrahltem UV-Licht zu reflektiertem und/oder emittiertem und/oder transmittierten und/oder gestreutem UV-Licht bestimmt. Mit Hilfe des spektroskopischen Kalibriermodells wird dem erhaltenen Verhältnis eine fiktive Schädigungszeit bei einer Temperatur von 200 °C zugeordnet. Dies kann beispielsweise mit einer App/Software auf einem Tablet, einem Laptop oder einem Smartphone erfolgen.

Die fiktiven Haarschädigungszeiten werden durch die App/Software folgende thermische Haarschädigungsgrade eingeordnet:

| Fiktive Haarschädigungszeit bei 200 °C | thermischer Haarschädigungsgrad |
|---|---|
| bis 10 Minuten | gering |
| 10 bis 30 Minuten | mäßig |
| 30 bis 60 Minuten | stark |
| größer 60 Minuten | sehr stark |

Entsprechend des thermischen Haarschädigungsgrad ermittelt die App/Software eine Blondiermittelempfehlung und/oder Haarfärbemittelempfehlung und/oder Haarpflegeproduktempfehlung und/oder Haarstylingproduktempfehlung.

Je nach thermischen Haarschädigungsgrad sollten die empfohlenen Blondiermittel und/oder Haarfärbemittel und/oder Haarpflegeprodukte und/oder Haarstylingprodukte folgende Inhaltsstoffe enthalten:

| thermischer Haarschädigungsgrad | Produktempfehlung |
|---|---|
| gering | Produkt enthält bis zu 0,5 Gew.-% Polysiloxan, insbesondere PEG-14 Dimethicone (INCI) |
| mäßig | Produkt enthält bis zu 0,5 Gew.-% Polysiloxan, insbesondere PEG-14 Dimethicone, und bis zu 0,5 Gew.-% filmbildendes Polymer, insbesondere Octylacrylamide / Acrylates / Butylaminoethyl Methacrylate Copolymer (INCI) |
| stark | Produkt enthält bis zu 1 Gew.-% Polysiloxan, insbesondere PEG-14 Dimethicone, und bis zu 0,5 Gew.-% filmbildendes Polymer, insbesondere Octylacrylamide / Acrylates / Butylaminoethyl Methacrylate Copolymer (INCI) |
| sehr stark | Produkt enthält bis zu 2 Gew.-% Polysiloxan, insbesondere PEG-14 Dimethicone, und bis zu 1 Gew.-% filmbildendes Polymer, insbesondere Octylacrylamide / Acrylates / Butylaminoethyl Methacrylate Copolymer (INCI) |

Beispiel 2 außerhalb des Umfangs der angehängten Ansprüche, jedoch hilfreich für das Verständnis der Ansprüche

### 1. Spektroskopisches Kalibriermodell ("Trypthophangehalt")

Zum Ermitteln eines spektroskopischen Kalibriermodells werden zunächst Haarsträhnen mit verschiedenen thermischen Schädigungsgraden (gering, mäßig, stark und sehr stark) jeweils mit UV-Licht mit einer Wellenlänge von 270 bis 290 nm bestrahlt. Das Licht, welches mit den Haarsträhnen gewechselwirkt hat, wird in einem Wellenlängenbereich von 270 bis 290 nm detektiert. Anschließend wird für diesen Bereich das Verhältnis von eingestrahlten zu detektiertem UV-Licht bestimmt. Dadurch wird ein, für den jeweiligen thermischen Schädigungsgrad charakteristisches UV-Absorptionsspektrum erhalten. Die für jeden thermischen Schädigungsgrad erhaltenen Spektren werden gemittelt.

Anschließend werden jeweils zehn Haarsträhnen eines thermischen Schädigungsgrades nach einem basischen Aufschluss mittels Hochleistungsflüssigkeitschromatographie (HPLC) untersucht und der Gehalt an Trypthophan bestimmt. Die erhaltenen Werte für jeden thermischen Schädigungsgrad werden gemittelt.

Dem für jeden thermischen Schädigungsgrad gemittelten Tryptophangehalt wird das entsprechende gemittelte Absorptionsspektrum zugeordnet.

### 2. Bestimmung des thermischen Haarschädigungsgrad

Zur Bestimmung einer individuellen thermischen Haarschädigung wird mindestens eine Haarsträhne, umfassend rund 100 Haare, eines Individuums mit UV-Licht mit einer Wellenlänge von 270 bis 290 nm bestrahlt und das Licht, welches mit den Haarsträhnen gewechselwirkt hat, in einem Wellenlängenbereich von 270 bis 290 nm detektiert. Mit Hilfe des spektroskopischen Kalibriermodells wird dem erhaltenen Absorptionsspektrum ein Trypthophangehalt zugeordnet. Dies kann beispielsweise mit einer App/Software auf einem Tablet, einem Laptop oder einem Smartphone erfolgen.

Entsprechend des Gehalts an Trypthophan ermittelt die App/Software eine Blondiermittelempfehlung und/oder Haarfärbemittelempfehlung und/oder Haarpflegeproduktempfehlung und/oder Haarstylingproduktempfehlung.

## Patentansprüche

1. Verfahren zum Ermitteln eines thermischen Schädigungsgrads von Haar (102), aufweisend:
während eines Belichtens einer Haarprobe (102P) des Haars mit UV- oder UV/VIS-Licht (110), Aufnehmen eines Spektrums von zumindest einem Teil des UV- oder UV/VIS-Lichts, welches mit der Haarprobe gewechselwirkt hat;
Vergleichen zumindest eines Teils des Spektrums mit einem mittels UV- oder UV/VIS-Spektren und thermischen Schädigungsgraden einer Mehrzahl von Kalibrierhaarproben gewonnenen spektroskopischen Kalibriermodell; und
Ermitteln des thermischen Schädigungsgrads des Haars unter Einbeziehung des Vergleichs, wobei das Verfahren ferner aufweist:
Erstellen des Kalibriermodells, aufweisend:
für die Mehrzahl von Kalibrierhaarproben:
während eines Belichtens der Kalibrierhaarprobe mit UV- oder UV/VIS-Licht,
Aufnehmen eines Spektrums von zumindest einem Teil des UV- oder UV/VIS-Lichts, welches mit der Kalibrierhaarprobe gewechselwirkt hat;
Ermitteln eines thermischen Schädigungsgrads der Kalibrierhaarprobe mittels eines unabhängigen Verfahrens; und
Zuweisen des thermischen Schädigungsgrads zum Kalibrierspektrum; und
Ermitteln einer Korrelation zwischen der Mehrzahl von Kalibrierspektren und der Mehrzahl von thermischen Schädigungsgraden.

2. Verfahren gemäß Anspruch 1,
wobei das unabhängige Verfahren ein Ermitteln eines Tryptophangehalts aufweist.

3. Verfahren gemäß Anspruch 2,
wobei das Ermitteln des Tryptophangehalts ein chromatographisches oder colorimetrisches Verfahren aufweist.

4. Verfahren gemäß einem der Ansprüche 1 bis 3,
wobei der zumindest eine Teil des UV- oder UV/VIS-Lichts einen Wellenlängenbereich um etwa 280 nm, vorzugsweise zwischen 270 und 290 nm, aufweist.

5. Verfahren gemäß einem der Ansprüche 1 bis 4,
wobei das Belichten der Haarprobe und/oder der Kalibrierhaarprobe mit UV- oder UV/VISmit einem Wellenlängenbereich um etwa 280 nm, vorzugsweise zwischen 270 und 290 nm, erfolgt.

6. Verfahren gemäß Anspruch 1, ferner aufweisend:
Für das Erstellen des Kalibriermodells,
für die Mehrzahl von Kalibrierhaarproben:
Ermitteln eines Intensitätsverhältnisses der Intensität an UV- oder UV/VIS-Licht mit dem die Kalibrierhaarproben belichtet wurden zu im Spektrum detektierter Intensität an UV- oder UV/VIS-Licht für jede detektierte Wellenlänge.

7. Verfahren gemäß Anspruch 1,
wobei das unabhängige Verfahren ein Ermitteln einer fiktiven Haarschädigungszeit aufweist.

8. Verfahren gemäß einem der Ansprüche 5 oder 7,
wobei der zumindest eine Teil des UV- oder UV/VIS-Lichts einen Wellenlängenbereich von 300 bis 350 nm aufweist.

9. Verfahren gemäß einem der Ansprüche 7 oder 8,
wobei das Belichten der Haarprobe und/oder der Kalibrierhaarprobe mit UV- oder UV/VIS-Licht einer Wellenlänge von 250 bis 300 nm erfolgt.

10. Vorrichtung zum Ermitteln eines thermischen Schädigungsgrads von Haar (102), aufweisend:
eine UV-Lichtquelle (106) oder einer UV/VIS-Lichtquelle zum Belichten einer Haarprobe (102P) des Haars mit UV-oder UV/VIS-Licht (110);
ein Spektrometer (108) zum Aufnehmen eines Spektrums von zumindest einem Teil des UV-oder UV/VIS-Lichts, welches mit der Haarprobe gewechselwirkt hat; und zum Aufnehmen eines Kalibrierspektrums von zumindest einem Teil des UV- oder UV/VIS-Lichts, welches mit einer Kalibrierhaarprobe gewechselwirkt hat für eine Mehrzahl von Kalibrierhaarproben, während eines Belichtens der Kalibrierhaarprobe mit dem UV- oder UV/VIS-Licht; und
eine Datenverarbeitungsvorrichtung (116) mit einem Datenspeicher, in welchem ein mittels UV- oder UV/VIS-Spektren und thermischen Schädigungsgraden einer Mehrzahl von Kalibrierhaarproben gewonnenes spektroskopisches Kalibriermodell gespeichert ist, und mit einem Prozessor zum Vergleichen zumindest eines Teils des Spektrums mit dem spektroskopischen Kalibriermodell und zum Ermitteln des thermischen Schädigungsgrads des Haars unter Einbeziehung des Vergleichs, wobei das in der Datenverarbeitungsvorrichtung gespeicherte Kalibriermodel wie folgt erstellt ist:
für die Mehrzahl von Kalibrierhaarproben:
während eines Belichtens der Kalibrierhaarprobe mit UV- oder UV/VIS-Licht,
Aufnehmen eines Spektrums von zumindest einem Teil des UV- oder UV/VIS-Lichts, welches mit der Kalibrierhaarprobe gewechselwirkt hat;
Ermittelt eines thermischen Schädigungsgrads der Kalibrierhaarprobe mittels eines unabhängigen Verfahrens; und
Zuweisen des thermischen Schädigungsgrads zum Kalibrierspektrum; und
Ermitteln einer Korrelation zwischen der Mehrzahl von Kalibrierspektren und der Mehrzahl von thermischen Schädigungsgraden.

11. Vorrichtung gemäß Anspruch 10,
wobei die UV-oder UV/VIS-Lichtquelle und das Spektrometer eine integrierte Einheit bilden.

12. Vorrichtung gemäß einem der Ansprüche 10 oder 11,
wobei die integrierte Einheit und/oder die Datenverarbeitungsvorrichtung mobile Geräte sind.

13. Verfahren zum Ermitteln einer individuellen Behandlungsanweisung, aufweisend:
Ermitteln eines thermischen Schädigungsgrads von Haar eines Individuums gemäß einem der Ansprüche 1 bis 9; und
computergestütztes Ermitteln einer individuellen Behandlungsanweisung in Abhängigkeit vom ermittelten thermischen Schädigungsgrad.

14. Verfahren gemäß Anspruch 13,
wobei die individuelle Behandlungsanweisung die Empfehlung von Blondiermitteln und/oder Haarfärbemitteln und/oder Haarpflegeprodukten und/oder Haarstylingprodukten umfasst.

## Claims

1. A method for determining a degree of heat damage to hair (102), comprising:
while exposing a hair sample (102P) of the hair to UV or UV/VIS light (110), recording a spectrum of at least one part of the UV or UV/VIS light that has interacted with the hair sample;
comparing at least one part of the spectrum with a spectroscopic calibration model obtained by means of UV or UV/VIS spectra and degrees of heat damage to a plurality of calibration hair samples; and
determining the degree of heat damage to the hair taking into account the comparison, wherein the method further comprises:
creating the calibration model, comprising:
for the plurality of calibration hair samples:
while exposing the calibration hair sample to UV or UV/VIS light,
recording a spectrum of at least one part of the UV or VIS light that has interacted with the hair sample;
determining a degree of heat damage to the calibration hair sample using an independent method; and
assigning the degree of heat damage to the calibration spectrum; and
determining a correlation between the plurality of calibration spectra and the plurality of degrees of heat damage.

2. The method according to claim 1,
wherein the independent method comprises determining a tryptophan content.

3. The method according to claim 2,
wherein determining the tryptophan content comprises a chromatographic or colorimetric method.

4. The method according to one of claims 1 to 3,
wherein the at least one part of the UV or UV/VIS light has a wavelength range of approximately 280 nm, preferably between 270 and 290 nm.

5. The method according to one of claims 1 to 4,
wherein the hair sample and/or the calibration hair sample is exposed to UV or UV/VIS light in a wavelength range of approximately 280 nm, preferably between 270 and 290 nm.

6. The method according to claim 1, further comprising:
for creating the calibration model,
for the plurality of calibration hair samples:
determining an intensity ratio of the intensity of UV or UV/VIS light at which the calibration hair samples were exposed to the intensity of UV or UV/VIS light detected in the spectrum for each detected wavelength.

7. The method according to claim 1,
wherein the independent method comprises determining a hypothetical hair damage time.

8. The method according to one of claims 5 or 7,
wherein the at least one part of the UV or UV/VIS light has a wavelength range of 300 to 350 nm.

9. The method according to one of claims 7 or 8,
wherein the hair sample and/or the calibration hair sample is exposed to UV or UV/VIS light having a wavelength of 250 to 300 nm.

10. A device for determining a degree of heat damage to hair (102), comprising:
a UV light source (106) and/or a UV/VIS light source for exposing a hair sample (102P) of the hair to UV or UV/VIS light (110);
a spectrometer (108) for recording a spectrum of at least one part of the UV or UV/VIS light that has interacted with the hair sample; and for recording a calibration spectrum of at least one part of the UV or UV/VIS light that has interacted with a calibration hair sample, for a plurality of calibration hair samples, while exposing the calibration hair sample to the UV or UV/VIS light; and
a data processing device (116) having a data memory in which a spectroscopic calibration model obtained by means of UV or UV/VIS spectra and degrees of heat damage to a plurality of calibration hair samples is stored, and having a processor for comparing at least one part of the spectrum with the spectroscopic calibration model and for determining the degree of heat damage to the hair taking into account the comparison, wherein the calibration model stored in the data processing device is created as follows:
for the plurality of calibration hair samples:
while exposing the calibration hair sample to UV or UV/VIS light,
recording a spectrum of at least one part of the UV or VIS light that has interacted with the hair sample;
determining a degree of heat damage to the calibration hair sample using an independent analytical method; and
assigning the degree of heat damage to the calibration spectrum; and
determining a correlation between the plurality of calibration spectra and the plurality of degrees of heat damage.

11. The device according to claim 10,
wherein the UV or UV/VIS light source and the spectrometer form an integrated unit.

12. The method according to one of claims 10 or 11,
wherein the integrated unit and/or the data processing device are mobile apparatuses.

13. A method for determining an individual treatment recommendation, comprising:
determining a degree of heat damage to the hair of an individual according to one of claims 1 to 9; and
determining, with the aid of a computer, an individual treatment recommendation depending on the degree of heat damage determined.

14. The method according to claim 13,
wherein the individual treatment recommendation comprises recommending bleaching agents and/or hair coloring agents and/or hair care products and/or hair styling products.

## Revendications

1. Procédé permettant de déterminer un degré d'endommagement thermique de cheveux (102), présentant :
pendant une exposition d'un échantillon capillaire (102P) des cheveux à une lumière UV ou UV-Visible (110), l'enregistrement d'un spectre d'au moins une partie de la lumière UV ou UV-Visible qui a interagi avec l'échantillon capillaire ;
la comparaison d'au moins une partie du spectre avec un modèle d'étalonnage spectroscopique obtenu au moyen de spectres UV ou UV-Visibles et de degrés d'endommagement thermique d'une pluralité d'échantillons capillaires d'étalonnage ; et
la détermination du degré d'endommagement thermique des cheveux en prenant en compte la comparaison, dans lequel le procédé présente en outre :
la création du modèle d'étalonnage, présentant :
pour la pluralité d'échantillons capillaires d'étalonnage :
pendant une exposition de l'échantillon capillaire d'étalonnage à la lumière UV ou UV-Visible,
l'enregistrement d'un spectre d'au moins une partie de la lumière UV ou UV-Visible qui a interagi avec l'échantillon capillaire d'étalonnage ;
la détermination d'un degré d'endommagement thermique de l'échantillon capillaire d'étalonnage au moyen d'un procédé indépendant ; et
l'attribution du degré d'endommagement thermique au spectre d'étalonnage ; et
la détermination d'une corrélation entre la pluralité de spectres d'étalonnage et la pluralité de degrés d'endommagement thermique.

2. Procédé selon la revendication 1,
dans lequel le procédé indépendant présente la détermination d'une teneur en tryptophane.

3. Procédé selon la revendication 2,
dans lequel la détermination de la teneur en tryptophane présente un procédé chromatographique ou colorimétrique.

4. Procédé selon l'une des revendications 1 à 3,
dans lequel l'au moins une partie de la lumière UV ou UV-Visible présente une gamme de longueurs d'ondes d'environ 280 nm, de préférence comprise entre 270 et 290 nm.

5. Procédé selon l'une des revendications 1 à 4,
dans lequel l'exposition de l'échantillon capillaire et/ou de l'échantillon capillaire d'étalonnage à des UV ou à des UV-Visibles est effectuée avec une gamme de longueurs d'ondes d'environ 280 nm, de préférence comprise entre 270 et 290 nm.

6. Procédé selon la revendication 1, présentant en outre :
pour la création du modèle d'étalonnage,
pour la pluralité d'échantillons capillaires d'étalonnage :
la détermination d'un rapport d'intensité entre l'intensité de la lumière UV ou UV-Visible à laquelle les échantillons capillaires d'étalonnage ont été exposés et l'intensité de la lumière UV ou UV-Visible détectée dans le spectre pour chaque longueur d'onde détectée.

7. Procédé selon la revendication 1,
dans lequel le procédé indépendant présente la détermination d'une durée d'endommagement capillaire fictive.

8. Procédé selon l'une des revendications 5 ou 7,
dans lequel l'au moins une partie de la lumière UV ou UV-Visible présente une gamme de longueurs d'ondes allant de 300 à 350 nm.

9. Procédé selon l'une des revendications 7 ou 8,
dans lequel l'exposition de l'échantillon capillaire et/ou de l'échantillon capillaire d'étalonnage est effectuée avec une lumière UV ou UV-Visible d'une longueur d'onde allant de 250 à 300 nm.

10. Dispositif permettant de déterminer un degré d'endommagement thermique de cheveux (102), présentant :
une source de lumière UV (106) ou une source de lumière UV-Visible pour l'exposition d'un échantillon capillaire (102P) des cheveux à la lumière UV ou UV-Visible (110) ;
un spectromètre (108) pour l'enregistrement d'un spectre d'au moins une partie de la lumière UV ou UV-Visible qui a interagi avec l'échantillon capillaire ; et pour l'enregistrement d'un spectre d'étalonnage d'au moins une partie de la lumière UV ou UV-Visible qui a interagi avec un échantillon capillaire d'étalonnage pour une pluralité d'échantillons capillaires d'étalonnage, pendant une exposition de l'échantillon capillaire d'étalonnage à la lumière UV ou UV-Visible ; et
un dispositif de traitement de données (116) comportant une mémoire de données dans laquelle est mémorisé un modèle d'étalonnage spectroscopique obtenu au moyen de spectres UV ou UV-Visibles et de degrés d'endommagement thermique d'une pluralité d'échantillons capillaires d'étalonnage, et comportant un processeur pour la comparaison d'au moins une partie du spectre avec le modèle d'étalonnage spectroscopique et pour la détermination du degré d'endommagement thermique des cheveux en prenant en compte la comparaison, dans lequel le modèle d'étalonnage mémorisé dans le dispositif de traitement de données est créé comme suit :
pour la pluralité d'échantillons capillaires d'étalonnage :
pendant une exposition de l'échantillon capillaire d'étalonnage à la lumière UV ou UV-Visible,
enregistrement d'un spectre d'au moins une partie de la lumière UV ou UV-Visible qui a interagi avec l'échantillon capillaire d'étalonnage ;
détermination d'un degré d'endommagement thermique de l'échantillon capillaire d'étalonnage au moyen d'un procédé indépendant ; et
attribution du degré d'endommagement thermique au spectre d'étalonnage ; et
détermination d'une corrélation entre la pluralité de spectres d'étalonnage et la pluralité de degrés d'endommagement thermique.

11. Dispositif selon la revendication 10,
dans lequel la source de lumière UV ou UV-Visible et le spectromètre forment une unité intégrée.

12. Dispositif selon l'une des revendications 10 ou 11,
dans lequel l'unité intégrée et/ou le dispositif de traitement de données sont des appareils mobiles.

13. Procédé permettant de déterminer une instruction de traitement individuelle, présentant :
la détermination d'un degré d'endommagement thermique de cheveux d'un individu selon l'une des revendications 1 à 9 ; et
la détermination assistée par ordinateur d'une instruction de traitement individuelle en fonction du degré d'endommagement thermique déterminé.

14. Procédé selon la revendication 13,
dans lequel l'instruction de traitement individuelle comprend la recommandation d'agents décolorants et/ou d'agents de coloration capillaire et/ou de produits de soins capillaires et/ou de produits coiffants.
